(19) Europäisches Patentamt
European Patent Office
Office européen des brevets

(11) Publication number: **0 431 605 A2**

# (12) EUROPEAN PATENT APPLICATION

(21) Application number: **90123349.4**

(22) Date of filing: **05.12.90**

(51) Int. Cl.5: **C07D 487/04**, C07D 471/04, //(C07D471/04,221:00,209:00)

(30) Priority: **05.12.89 JP 314312/89**

(43) Date of publication of application: **12.06.91 Bulletin 91/24**

(84) Designated Contracting States: **AT BE CH DE DK ES FR GB GR IT LI LU NL SE**

(71) Applicant: **SANWA KAGAKU KENKYUSHO CO., LTD.**
**No. 35, Higashi-sotobori-cho, Higashi-ku Nagoya, Aichi-ken(JP)**

(72) Inventor: **Kurono, Masayasu, Sanwa Kagaku Kenkyusho Co., Ltd.**
**35, Higashi-sotobori-cho, Higashi-ku Nagoya, Aichi-ken(JP)**
Inventor: **Kondo, Yasuaki, Sanwa Kagaku Kenkyusho Co., Ltd.**
**35, Higashi-sotobori-cho, Higashi-ku Nagoya, Aichi-ken(JP)**
Inventor: **Unno, Ryoichi, Sanwa Kagaku Kenkyusho Co., Ltd.**
**35, Higashi-sotobori-cho, Higashi-ku Nagoya, Aichi-ken(JP)**
Inventor: **Matsumoto, Yukiharu, Sanwa Kagaku Kenkyusho Co Ltd**
**35, Higashi-sotobori-cho, Higashi-ku Nagoya, Aichi-ken(JP)**
Inventor: **Oka, Mitsuru, Sanwa Kagaku Kenkyusho Co., Ltd.**
**35, Higashi-sotobori-cho, Higashi-ku Nagoya, Aichi-ken(JP)**
Inventor: **Baba, Kunihisa, Sanwa Kagaku Kenkyusho Co., Ltd.**
**35, Higashi-sotobori-cho, Higashi-ku Nagoya, Aichi-ken(JP)**
Inventor: **Sawai, Kiichi, Sanwa Kagaku Kenkyusho Co., Ltd.**
**35, Higashi-sotobori-cho, Higashi-ku Nagoya, Aichi-ken(JP)**

(74) Representative: **Wächtershäuser, Günter, Dr.**
**Tal 29**
**W-8000 München 2(DE)**

## (54) 1-Azabicyclo[m.n.O]alkane derivatives, their salts and process for preparing them.

(57) The invention is directerd to a 1-azabicyclo[m.n.O]alkane derivative of the formula

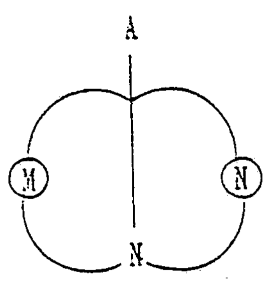

(I)

wherein M represents a Cm alkylene chain which may be substituted by 1 - 2m hydrocarbon groups, N represents Cn alkylene chain which may be substituted by 1 - 2n hydrocarbon groups, m and n are an integer of 3 - 5 (m > n), A represents a cyano radical, carboxylic acid group which has been esterified with an alcohol, a carboxylic acid group which has been amidized with a primary or secondary amine, an acyl group, or radical of $-C(A^1)(A^2)(A^3)$, in which at least one of $A^1$, $A^2$ and $A^3$ is a nitro radical, hydroxyl radical, hydroxyl

EP 0 431 605 A2

group etherified with an alcohol or esterified with a carboxylic acid, unsubstituted or mono- or di-substituted amino group, acylated amino group or halogen atom, and the others of $A^1$, $A^2$ and $A^3$ are hydrogen or a hydrocarbon group,

a salt thereof as well as a process for the preparation thereof. The derivative is used as an intermediate for preparing medicines for animals and agricultural chemicals.

# 1-AZABICYCLO[M.N.O]ALKANE DERIVATIVES, THEIR SALTS AND PROCESS FOR PREPARING THEM

The present invention relates to a 1-azabicyclo [m.n.O]alkane derivative, a salt thereof and a process for the preparation of the same.

Some of 1-azabicyclo[m,n,O]alkane derivatives are contained per se in the partial structure of the basic skeleton of alkaloids. The 1-azabicyclo[m.n.O]alkane derivatives form in turn the partial structures of the pharmacologically active substances developed by the present inventors such as 2-oxopyrrolidine compounds [Jap. Pat. No. Sho 61 - 254587(A)], organo platinum complexes [Jap. Pat. No. Sho 61 - 229893(A)] and cephalosporin derivatives [Jap. Pat. No. Sho 62 - 16487(A)], and can be also used as an intermediate for preparing various medicines for animals and agricultural chemicals.

There are very few known (m + 2)-position substituted-1-azabicyclo[m.n.O]alkane derivatives, in which m > n and substituents are present at positions other than the (m + 2)-position on the ring. Though the following methods are known as the methods for synthesizing (m + 2)-position-substituted-1-azabicyclo-[m.n.O]alkane derivatives, none of these methods can be widely employed as general one to easily synthesize various analogous compounds.

That is, when 5-position-substituted-1-azabicyclo [3.3.0]-octane derivatives are synthesized, first of all γ -butyrolactone is reacted with KOCN to give γ -(N-2-pyrrolidinonyl) butyric acid, which is, in turn, heated in the presence of soda lime to form 1-azabicyclo[3.3.0]-4-octene. The 1-azabicyclo compound is further reacted with perchloric acid to form 1-azabicyclo[3.3.0]octa-$\Delta^{1(e)}$-nium perchlorate, which is then reacted with a nucleophilic reagent such as potassium cyanate, cyanoacetic acid or a substituted malonic acid derivative to perform chemical transformation of the introduced functional radical, if necessary and to give the 5-position-substituted-1-azabicyclo[3.3.0]octane derivatives [MIYANO et al., "Synthesis", page 701 (1978); MIYANO et al., "Heterocycles" Vol. 16, page 755 (1981); Jap. Pat. Nos. Sho 59 - 112988(A), 59 - 167591(A) and 61 - 22084(A)]. In addition, as the method for synthesizing γ -(N-2-pyrrolidinonyl)butyric acid as the intermediate for the aforementioned synthetic method, there has been also reported a method for reacting 2-pyrrolidone with metallic sodium followed by adding γ -butyrolactone to the reaction mixture [MIYANO et al., "J. Heterocyclic Chem.", Vol.19, page 1465 (1982)].

When 6-position-substituted-1-azabicyclo[4.3.0]nonane derivatives are synthesized, first of all 2-piperidone is reacted with metallic sodium, and the reaction mixture is, in turn, reacted with γ -butyrolactone to give γ - (N-2-piperidinonyl)butyric acid. The butyric acid derivative is heated in the presence of soda lime in the same manner as the aforementioned 5-position-substituted-1-azabicyclo[3,3,0]octane derivative and then reacted with perchloric acid to give 1-azabicyclo[4.3.0]nona-$\Delta^{1(e)}$-nium perchlorate. The perchlorate is next reacted with a nucleophilic reagent such as potassium cyanate, cyanoacetic acid or a substituted malonic acid derivative and led to a desired compound, if necessary, by chemical transformation of the introduced functional radical [MIYANO et al., "J. Heterocyclic Chem.", Vol. 19, page 1465 (1982)].

When 6-position-substituted-1-azabicyclo[4,4,0]decane derivatives are synthesized, first of all 2-piperidone is reacted with potassium hydride, and the reaction mixture is, in turn, reacted with ethyl 5-bromovalerate to give ethyl γ -(N-2-piperidinonyl) valerate. The valerate derivative is heated in the presence of soda lime in the same manner as the aforementioned 5-posiition-substituted-1-azabicyclo[3.3.0]octane derivative and then reacted with tetrafluoroboric acid to give 1-azabicyclo[4.3.0]nona-$\Delta^{1(e)}$-nium tetrafluoroborate. The tetrafluoroborate derivative is then reacted with a nucleophilic reagent such as potassium cyanate, cyanoacetic acid or a substituted malonic acid derivative and led to a desired compound, if necessary, by chemical transformation of the introduced functional radical [Roger W. Alder et al., "J. Chem. Soc., Chem, Commun.", page 940 (1982)].

Furthermore, there are disclosed 5-nitromethyl-1-azabicyclo[3.3.0]octane, 5-cyano-1-azabicyclo[3.3.0]-octane, 5-aminomethyl-1-azabicyclo[3.3.0]octane in Jap. Pat. Nos. Sho 63 - 138405(A) and 63 - 138406(A).

The first step of the aforementioned conventional reaction process as the step for synthesizing γ - (N-2-pyrrolidononyl)butyric acid has disadvantages of that high temperature (about 200 °C ) is required for the reaction, and that the reaction has a relatively low efficiency (Yield of about 40%). On the other hand, the reaction comprising reacting 2-pyrrolidone or 2-piperidone with metallic sodium or potassium hydride and adding γ -butyrolactone or 5-bromovaleric acid to the reaction mixture has a disadvantage of that there is a possibility of abnormal reaction such as explosion or the like.

The second step of the aforementioned conventional reaction process as the step for heating γ -(N-2-pyrrolidinonyl)butyric acid, γ - (N-2-piperidinonyl)butyric acid or γ -(N-2-piperidinonyl)valeric acid in the presence of soda lime and synthesizing 1-azabicyclo[4.3.0]-4-octene, 1-azabicyclo[4.3.0]-5-nonene or 1-azabicyclo[4.4.0]-5-decene has disadvantages of that high temperature (200-300 °C) is required for the reaction and that the reaction product has a low stability.

In the Japanese Official Gazettes [Jap. Pat. Nos. Sho 63 - 138405 and 63 - 138406, there are given disclosures for preparing 5-nitromethyl-1-azabicyclo[3.3.0]octane, 5-cyano-1-azabicyclo[3.3.0]octane, 5-aminomethyl-1-azabicyclo[3.3.0]octane, but the Gazettes do not show a process for the preparation of other 1-azabicyclo[m.n.O]alkane derivatives.

Therefore, the first object of the invention is to provide 1-azabicyclo[m.n.O]alkane derivatives and salts thereof, which are useful as the raw materials for synthesizing various medicines for animals, agricultural chemicals and the like.

An additional but extremely important object of the invention is to provide novel 1-azabicyclo[m.n.O]-alkane derivatives and salts thereof which are different from conventional well-known 1-azabicyclo[m.n.O]-alkane derivatives in physico-chemical properties (partition coefficient, basicity, molecular refractive index, molecular steric conformation, etc.) and thus to bring a possibility of the improvement on pharmacological properties (improvement of activity, decrease of toxicity, etc.) or of discoveries of novel pharmacological actions in 2-oxopyrrolidine compounds [Jap. Pat. No. Sho 61 - 254587(A)], organo platinum complexes [Jap. Pat. No. Sho 61 - 229893(A)] and cephalosporin derivatives [Jap. Pat. No. Sho 62 - 16487(A)], which have been developed by the present inventors.

The second object of the invention is to provide a process for synthesizing a 1-azabicyclo[m.n.O]alkane derivative in an industrial scale, which has a wide uses suitable for the synthesis of various 1-azabicyclo-[m.n.O]alkane derivative, an easy controlling ability of reaction, no risk of explosion and no requirement of expensive reagents and thus is suitable for the production of 1-azabicyclo[m.n.O]alkane derivative in an industrial scale.

According to the invention, the aforementioned first object and its subsequent object can be attained by the 1-azabicyclo[m,n,O]alkane derivative of the formula

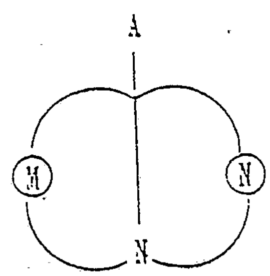

( I )

wherein M represents a Cm alkylene chain which may be substituted by 1 - 2m hydrocarbon groups, N represents Cn alkylene chain which may be substituted by 1 - 2n hydrocarbon groups, m and n are an integer of 3 - 5 (m > n), A represents a cyano radical, carboxylic acid group which has been esterified with an alcohol, a carboxylic acid group which has been amidized with a primary or secondary amine, an acyl group, or radical of $-C(A^1)(A^2)(A^3)$, in which at least one of $A^1$, $A^2$ and $A^3$ is a nitro radical, hydroxyl radical, hydroxyl group etherified with an alcohol or esterified with a carboxylic acid, unsubstituted or mono- or di-substituted amino group, acylated amino group or halogen atom, and the others of $A^1$, $A^2$ and $A^3$ are hydrogen or a hydrocarbon group,
and a salt thereof.

That is, the partition coefficient of the compound shown by formula (I), for example 5-cyano-4,6-dimethyl-1-azabicyclo-[3 .3 .0] octane increases to a extent of 1 .32 (theoretical) in comparison with 5-cyano-1-azabicyclo[3.3.0]octane as the unsubstituted derivative. When a pharmaceutical preparation containing the structure in its molecule is administered, it can be expected that its distribution in a living body changes to increase its pharmacological action and to express a certain novel pharmaceutical efficiency. Therefore, the compound shown by the formula (I) and a salt thereof are very useful as an intermediate for the synthesis of various medicines for animal and agricultural chemicals.

When the compound (I) has structurally asymmetric carbon atom(s), it can contain the stereoisomers. It should be noted that these stereoisomers are also included in the compounds according to the present invention.

In the compounds (I), the hydrocarbon group may be an alkyl group or an aryl group. As the straight chain alkyl groups, there may be mentioned those having 1 - 10 carbon atoms such as methyl, ethyl, n-propyl, n-butyl, n-pentyl, n-hexyl, n-decyl groups and the like. As the branched chain alkyl groups, there may be mentioned 1-propyl, 1-butyl, sec-butyl, tert-butyl, 1-pentyl groups and the like. As the cyclic alkyl groups, there may be mentioned those having 3 or more carbon atoms such as cyclopentyl, cyclobutyl,

cyclopentyl, cyclohexyl, cycloheptyl groups and the like. As the aryl groups, there are mentioned phenyl, tolyl, xylyl, mesyl groups and the like.

As the alcohols, there may be mentioned aliphatic alcohols such as methyl alcohol, ethyl alcohol, n-propyl alcohol, n-butyl alcohol, n-pentyl alcohol, n-hexyl alcohol, isopropyl alcohol, t-butyl alcohol, sec-butyl alcohol, i-pentyl alcohol, cyclopropyl alcohol, cyclobutyl alcohol, cyclopentyl alcohol, cyclohexyl alcohol and the like.

As the primary amines, there may be mentioned aliphatic amines such as methylamine, ethylamine, n-propylamine, n-butylamine, n-pentylamine, n-hexylamine, n-decylamine, isopropylamine, isobutylamine, sec-butylamine, tert-butylamine, i-pentylamine, cyclopropylamine, cyclobutylamine, cyclopentylamine, cyclohexylamine and the like, aromatic amines such as aniline and the like, heterocyclic amines such as pyridinylamine and the like.

As the secondary amines, there may be mentioned aliphatic amines such as dimethylamine, diethylamine, di-n-propylamine, morpholine, piperazine, piperidine, pyrrolidine and the like, and aromatic amines such as phenylbenzylamine, methoxyphenylbenzylamine and the like.

As the acyl groups, there may be mentioned aliphatic acyl groups such as acetyl, propionyl, butytyl, isobutyryl, valeryl, isovaleryl, pivaloyl and the like, and aromatic acyl groups such as benzoyl, cynnamoyl groups and the like.

As the monosubstituted amino groups, there may be mentioned aliphatic amino groups such as methylamino, ethylamino, n-propylamino, n-butylamino, n-pentylamino, n-hexylamine, n-decylamino, isopropylamino, isobutylamino, sec-butylamino, tert-butylamino, i-pentylamino, cyclopropylamino, cyclobutylamino, cyclopentylamino, cyclohexylamino groups and the like, aromatic groups such as anilino and the like, and heterocyclic amino groups such as a pyridinylamino group and the like.

As the di-substituted amino groups, there may be mentioned aliphatic amino groups such as dimethylamino, diethylamino, di-n-propylamino, morpholino, piperazino, piperidino, pyrrolidino and the like, and aromatic amino groups such as phenylbenzylamino, methoxyphenylbenzylamino groups and the like.

As the halogen atoms, there are mentioned chlorine, bromine and iodine.

As the salts of the compounds of the formula (I), which is of course non-toxic salts, there can be mentioned hydrogen halide salts such as hydrochloride, hydrobromide, hydroiodide, perchlorate and the like, mineral acid salts such as sulfate, nitrate, phosphate and the like, organic carboxylic acid salts such as acetate, propionate, glycolate, maleate, fumarate, tartrate, succinate, lactate, benzoate, cinnamate and the like, alkylsulfonates such as methanesulfonate and the like, arylsulfonates such as benzenesulfonate, p-toluenesulfonate and the like, cyclic alkylsulfonates such as cyclohexyl sulfonate and the like.

Among the compounds (I), the 1-azabicyclo[m.n.O]alkane derivatives of the formula

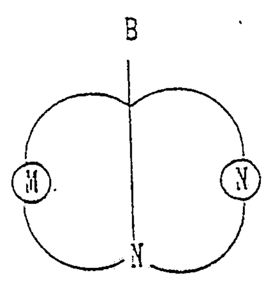

(V)

wherein M and N have the meanings as referred to, B represents a cyano radical or a group of $-C(B^1)(B^2)(B^3)$, at least one of $B^1$, $B^2$ and $B^3$ represents nitro groups, and the others represent hydrogen or a hydrocarbon group,

and a salt thereof can be prepared by reacting a α, ω -di-substituted alkanone of the formula

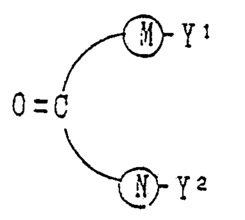

(II)

wherein M and N have the meanings as referred to, $Y^1$ and $Y^2$ represent a halogen atom or a group of

$R^1$-$SO_3$-, in which $R^1$ is a hydrocarbon group,
with ammonia and a cyano compound of the formula

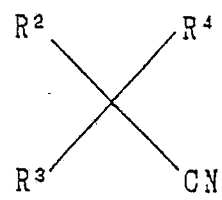

(III)

wherein $R^2$ and $R^3$ are same or different and represent hydrogen atom or a hydrocarbon group, respectively, and $R^4$ represents a hydroxyl or amino radical,
or a compound of the formula

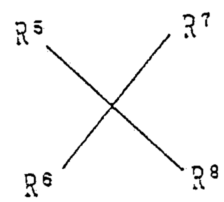

(IV)

wherein at least one of $R^5$, $R^6$, $R^7$ and $R^8$ is a nitro radical, and the others represent hydrogen atom or a hydrocarbon group,
and if necessary, converting resulting compound into the salt.

The compound thus obtained and represented by the formula (V) can be converted into the aforementioned compound represented by the formula (I) or a salt thereof by changing chemically the functional groups as usual and converting the compound into a salt, if necessary. Thus, the problems in the prior art can be solved to attain the aforesaid second object.

In the process for preparing the compound represented by the formula (V), the $\alpha$,$\omega$-di-substituted alkanone represented by the formula (II) as the raw material can be easily synthesized with use of methods known per se or applying the known methods. For example, 1,7-di-substituted-4-heptanone derivative can be synthesized by starting from $\gamma$-butyrolactone derivative and according to the method disclosed by H. Hart et al. ["J. Am. Chem. Soc.", Vol. 78, page 112 (1956)]. Alternatively, it can be easily synthesized by preparing 1,6-dioxaspiro[4.4]nonane derivative in accordance with the method described in the review by L. Yu. Brezhnev et al. ["Khim. Geterotsikl. Soedin.", page 1155 (1986), "Chem. Abstr.", Vol. 107, 115506q] and reacting the compound with hydrogen halide in accordance with the method disclosed by H. Hart et al. ["J. Am. Chem. Soc.", Vol. 78, page 112 (1956)]. A compound of 1,8-di-substituted-4-octanone derivative can be easily synthetized according to the method as disclosed by H. Stetter et al. ["Chem. Ber.", Vol. 91, page 2543 (1958)]. A compound of 1,9-di-substituted-4-octanone derivative can also be easily synthetized according to the method disclosed by H. Stetter et al. ["Chem. Ber.", Vol. 91, page 2543 (1958)], or it can be easily synthesized by applying the method as disclosed by H. Hart ["J. Am. Chem. Soc.", Vol. 78, page 112 (1956)], namely by using $\delta$-valerolactone derivative in place of $\gamma$-butyrolactone derivative.

In addition, among the compounds represented by the formula (III) as another raw material, cyanhydrin derivatives, wherein $R^4$ represents hydroxyl group, can be easily synthesized according to the method disclosed by Cox et al. ["Org. Syn.", Coll. II, page 7 (1966)], and aminonitrile derivatives can be easily synthesized according to the method as disclosed by R. A. Jacobson ["J. Am. Chem. Soc.", Vol. 68, 2628 (1946)] or according to the method disclosed In Jap. Pat. Nos. Sho 54 - 79232(A) and 61 - 87658(A). Also, as the compounds represented by the formula (IV) of the other raw material, there can be mentioned nitroalkane derivatives such as nitromethane, nitroethane and the like.

The synthesis of the compound represented by the formula (V) completes by stirring 1 - 100 equivalents of the compound represented by the formula (III) or (IV) and 3 - 50 equivalents of ammonia in proportion to 1 equivalent of the compound represented by the formula (II), in the presence or absence of a solvent at a temperature of 20 - 100°C for 5 - 100 hours. As the post-treatment method, it is possible to employ one of following two methods. The first method is that when an insoluble material presents in a reaction mixture, filtration is carried out, and the filtrate is concentrated in vacuo, and the resulting residue is

subjected to vacuum distillation or chromatography to isolate 1-azabicyclo[m.n.O]alkane derivative. In this first method, when an acid is added to the filtrate, the mixture is concentrated in vacuo and the resulting crystals are purified by recrystallization to obtain 1-azabicyclo[m.n.O]alkane derivative represented by the formula (V). Alternatively, a salt of 1-azabicyclo[m.n.O]alkane derivative represented by the formula (V) can also be obtained by dissolving 1-azabicyclo[m.n.O]alkane derivative isolated above in a solvent and then adding an acid to the solution. On the other hand, the second method is carried out by adding an alkali solution into the reaction mixture to make it alkaline, extracting the basic reaction mixture with an organic solvent, drying and concentrating the same in vacuo, and purifying through vacuum distillation or chromatography to isolate 1-azabicyclo[m.n.O]alkane derivative represented by the formula (V). The salt of the compound can be obtained in the same manner as described for the first method.

In the aforementioned reaction operation, ammonia can be added at the initiation of the reaction in all amount required for the reaction and by dissolving same into a solvent, or it can be added to the reaction mixture so that the amount of the ammonia is maintained at an amount of 0.5 - 5 equivalents in the reaction system or blown into the reaction mixture in the form of ammonia gas, or the reaction can be performed under the atmosphere of ammonia gas. It is also possible to carry out the reaction with a combination of these ammonia addition methods.

As the reaction solvent, there can be mentioned alcohols such as methanol, ethanol, n-propanol, 1-propanol and the like; hydrocarbon solvents such as n-hexane, cyclohexane, n-pentane, benzene, toluene, xylene and the like; and ether solvents such as ethyl ether, tetrahydrofuran and the like.

As the acid for the formation of the salt, there can be mentioned hydrogen halides such as hydrochloric acid, hydrobromic acid, hydroiodic acid, perchloric acid and the like; mineral acids such as sulfuric acid, nitric acid, phosphoric acid and the like; organic carboxylic acids such as acetic acid, propionic acid, glycolic acid, maleic acid, fumaric acid, tartaric acid, succinic acid, lactic acid, benzoic acid, cinnamic acid and the like; alkylsulfonic acids such as methanesulfonic acid and the like; arylsulfonic acids such as benzenesulfonic acid, p-toluenesulfonic acid and the like; and cyclic alkylsulfonic acids such as cyclohexylsulfonic acid and the like.

As the alkali solution, there can be used the aqueous solutions of basic materials such as sodium hydroxide, potassium hydroxide, sodium carbonate, potassium carbonate, potassium carbonate, sodium hydrogen carbonate and the like.

On the other hand, as the extraction solvents, there can be used organic solvents such as methylene chloride, chloroform, ethyl ether, ethyl acetate and the like.

In this connection, when stereoisomers are produced due to the presence of asymmetric carbons, isomers can be separated from each other through operations such as column chromatography, vacuum distillation, recrystallization or the like.

The invention will now be further explained in more detail with reference to Examples.

Example 1
6-Nitromethyl-1-azabicyclo[4.3.0]nonane hydrochloride

A mixture of 1,8-dibromo-4-octanone (500mg, 1.75 mmol) and nitromethane (10.7g, 175mmol) was stirred at 20° C under $NH_3$ atmosphere for 48hr. The reaction mixture was poured into ethyl ether saturated with hydrogen chloride. After evaporation of the solvent in vacuo, methylene chloride was added to the resulting residue and formed ammonium chloride was filtered off to concentrate a filtrate in vacuo. Resulting crystals were recrystallized from a mixture of chloroform and ethyl ether to afford the desired compound (200mg, Yield : 62.1%).

MS spectrum (EI/DI) m/z :
184 ($M^+$), 122 (base peak).
$^1$H-NMR spectrum (DMSO-$d_6$) δ ppm :

1.9 - 2.2,
3.2 - 3.4 and
3.6 - 3.7 (14H, m,

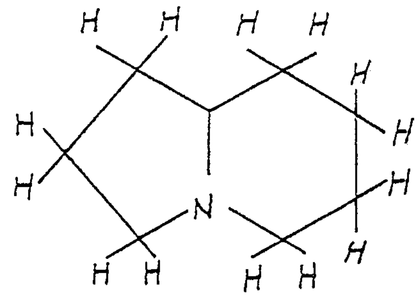

),

5.27 (1H, d, J = 12.2Hz, $CH_2NO_2$),
5.52 (1H, d, J = 12.2Hz, $CH_2NO_2$),
11.9 (1H, brs, $N^+H$).

IR spectrum ($\nu_{max}^{KBr}$) $cm^{-1}$ :
1550, 1380 ($NO_2$).

Example 2

5-Cyano-2,8-dimethyl-1-azabicyclo[3.3.0]octane

1,7-Dichloro-1,7-dimethyl-4-heptanone (250mg, 1.18mmol), 2-amino-2-methylpropanenitrile (310mg, 3.69mmol) and liquid ammonia (500mg, 29.4mmol) were charged in a sealed tube to stir at 50 - 60° C for 5hr. After cooling the reaction mixture, formed crystals were filtered off. The filtrate was concentrated in vacuo, and then the resulting crude product was purified by an alumina column chromatography (Merck No. 1097; elute : n-hexane/ EtOAc = 50/1) to afford the desired compound (81.6mg, Yield : 42.1%).

MS spectrum (EI/DI) m/z :
164 ($M^+$), 138 ($M^+$-CN), 124 (base peak).

$^1$H-NMR spectrum ($CDCl_3$) $\delta$ ppm :

1.18 (6H, d, J = 6.4Hz, 2 x Me),

1.75 - 1.82 (4H, m,

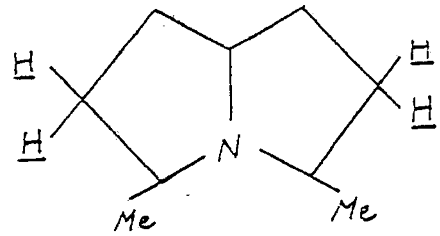

),

1.98 - 2.08 (2H, m,

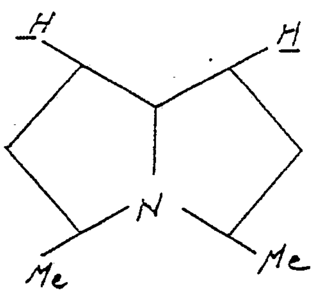

),

2.34 - 2.40 (2H, m,

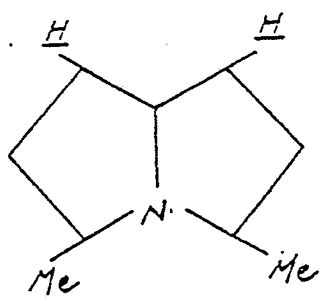

),

2.81 - 2.89 (2H, m,

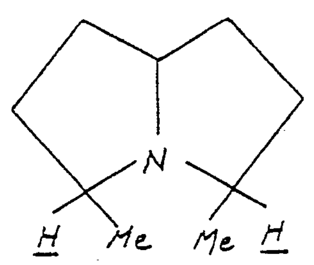

).

IR spectrum ($\nu_{max}^{neat}$) $cm^{-1}$ :
2938 (C-H), 2220 (CN).

Example 3

5-Cyano-4,6-dimethyl-1-azabicyclo[3.3.0]octane

1,7-Dichloro-3,5-dimethyl-4-heptanone (202mg, 0.957mmol), acetocyanhydrin (253mg, 2.97mmol) and were dissolved in liquid ammonia (400mg 23.5mmol) were charged in a sealed tube to stir at 50 - 60° C for

5hr. After cooling the reaction mixture, formed crystals were filtered off. The filtrate was concentrated in vacuo, and the resulting crude product was purified by an alumina column chromatography (Merck No. 1097; elute : n-hexane/EtOAc = 50/1) to afford the desired compound (82.3mg, Yield : 50.1%).

MS spectrum (EI/DI) m/z :

164 ($M^+$), 149 ($M^+$-Me), 136 (base peak).

$^1$H-NMR spectrum ($CDCl_3$) $\delta$ ppm :

1.25 (6H, d, J = 6.3Hz, 2 x Me),

1.84 - 2.13 (6H, m,

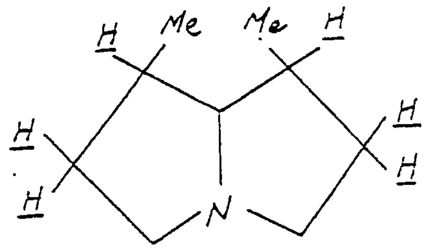

),

2.47 - 2.56 (2H, m,

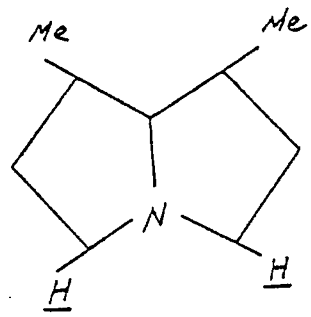

),

3.21 - 3.28 (2H, m,

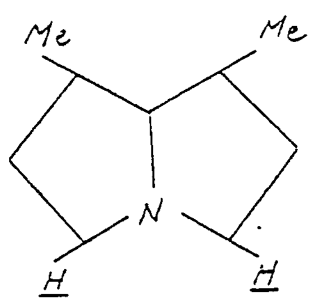

).

IR spectrum ($\nu_{max}^{neat}$) $cm^{-1}$ :

2938 (C-H) 2220 (CN).

Example 4

6-Cyano-1-azabicyclo[4.3.0]nonane

A mixture of 1,8-dibromo-4-octanone (500mg, 1.75 mmol) and 2-amino-2-methylpropanenitrile (460mg,

5.48mmol) was stirred under ammonia gas atmosphere at 20°C for 24hr. To the reaction mixture was added 0. 1N NaOH solution to extract with methylene chloride. The extract was dried over $Na_2SO_4$ and concentrated in vacuo. The resulting crude product was purified by an alumina column chromatography (Merck No. 1097; elute: n-hexane/EtOAc = 10/1) to afford the desired compound (150mg, Yield : 57.1%).

MS spectrum (EI/DI) m/z :

150 ($M^+$) , 122 (base peak).

$^1$H-NMR spectrum ($CDCl_3$) δ ppm :

1.5 - 1.9 (8H, m,

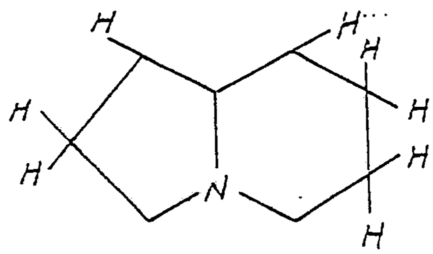

),

2.1 - 2.2 (2H, m,

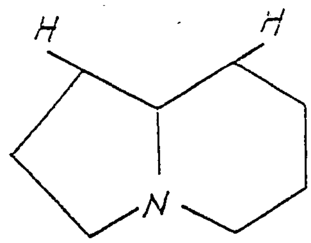

),

2.3 - 2.5 (2H, m,

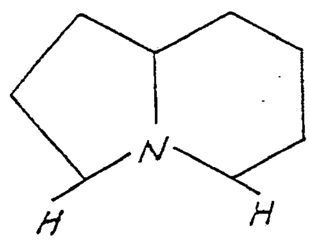

),

2.9 - 3.1 (2H, m,

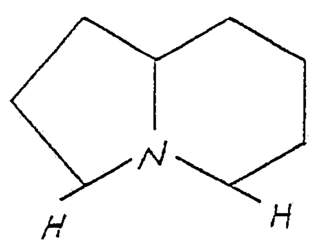

).

IR spectrum ($\nu_{max}^{neat}$) $cm^{-1}$ :
2938 (C-H) 2214 (CN).

Example 5

6-Cyano-1-azabicyclo[4.4.0]decane

1,9-Dichlorononan-5-one (300mg, 1.42mmol), 2-amino-2-methylpropanenitrile (380mg, 4.52mmol) and liquid ammonia (500mg, 29.4mmol) were charged in a sealed tube to stir at 50 - 60°C for 5hr. After cooling the reaction mixture, formed crystals were filtered off. The filtrate was concentrated in vacuo, and the resulting crude product was purified by an alumina column chromatography (Merck No. 1097; elute : n-hexane/EtOAc = 50/1) to afford the desired compound (131mg, Yield : 56.0%)

MS spectrum (EI/DI) m/z :
164 ($M^+$), 138 (base peak).
$^1H$-NMR spectrum ($CDCl_3$) $\delta$ ppm :

1.5 - 1.9 (12H, m,

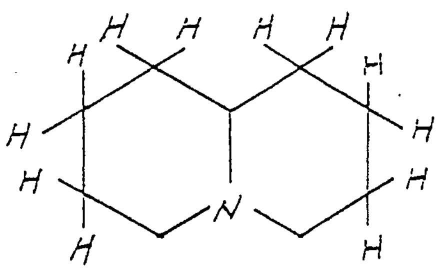

),

2.3 - 2.5 and
2.6 - 2.7 (4H, m,

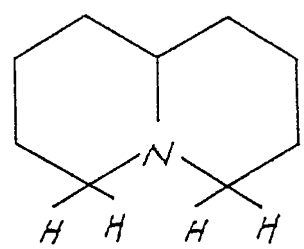

).

IR spectrum ($\nu_{max}^{neat}$) $cm^{-1}$ :
2938 (C-H) 2214 (CN).

Examples 6 - 10

Following compounds were prepared in the manner similar to those described In Example 1 to 5.

a) 5-Nitromethyl-2,8-dimethyl-1-azabicyclo[3.3.0]octane

Yield : 40.1%.
MS spectrum (EI/DI) m/z :
198 ($M^+$), 138 (base peak).
$^1H$-NMR spectrum ($CDCl_3$) $\delta$ ppm :

1.19 (6H, d, J = 6.4Hz, 2 x Me),

1.7 - 1.8 (4H, m,

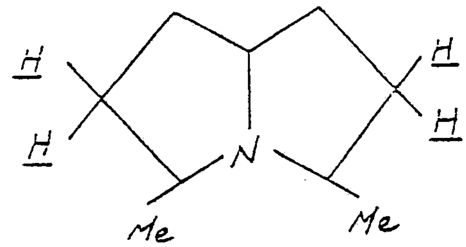

),

1.9 - 2.1 (2H, m,

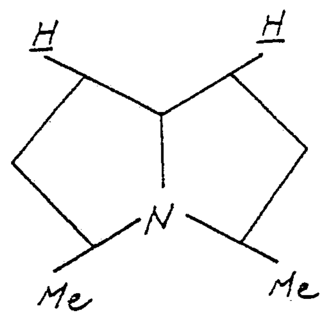

),

2.3 - 2.5 (2H, m,

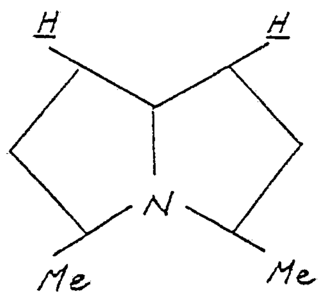

),

2.8 - 2.9 (2H, m,

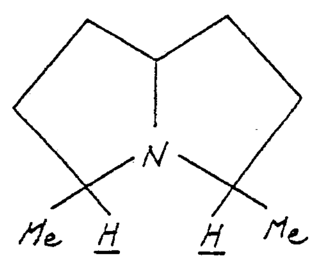

).

4.27 (2H, s, $CH_2NO_2$).

IR spectrum ($\nu_{max}^{neat}$) $cm^{-1}$ :
2940 (C-H), 1550, 1370 ($NO_2$).
b) 5-Nitromethyl-3,7-dimethyl-1-azabicyclo[3.3.0]octane
Yield : 52.9%.
MS spectrum (EI/DI) m/z :
198 ($M^+$), 138 (base peak).

$^1$H-NMR spectrum ($CDCl_3$) δ ppm :

1.10 (6H, d, J = 6.3Hz, 2 x Me),

1.7 - 1.8 (2H, m,

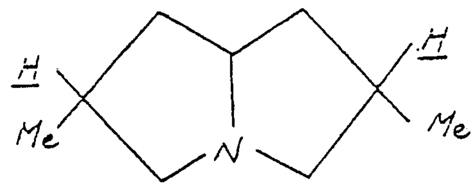

),

2.0 - 2.1 (2H, m,

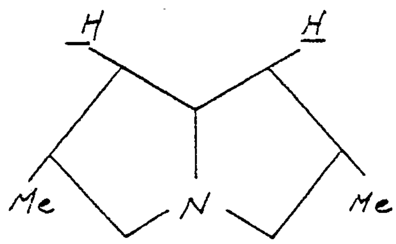

),

2.3 - 2.6 (4H, m,

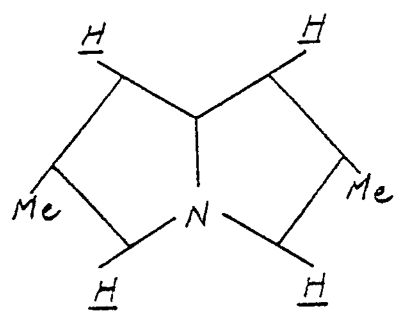

),

3.2 - 3.3 (2H, m,

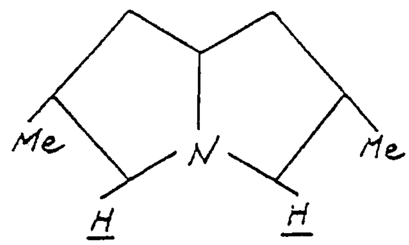

).

4.27 (2H, s, $CH_2NO_2$).

IR spectrum ($\nu_{max}^{neat}$) $cm^{-1}$ :
2930 (C-H), 1550, 1370 ($NO_2$).

c) <u>5-Nitromethyl-4,6-dimethyl-1-azabicyclo[3.3.0]octane</u>

Yield : 50.1%.
MS spectrum (EI/DI) m/z :
198 ($M^+$), 138 (base peak).
$^1$H-NMR spectrum ($CDCl_3$) $\delta$ ppm :

1.25 (6H, d, J = 6.3Hz, 2 x Me),

1.8 - 2.2 (6H, m,

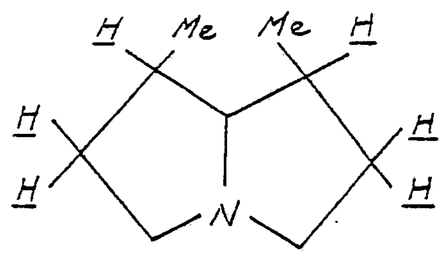

),

2.5 - 2.6 (2H, m,

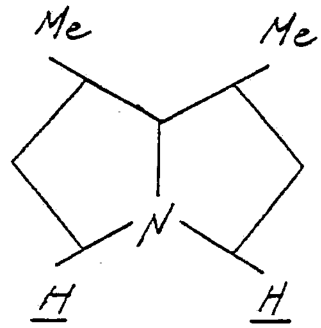

),

3.2 - 3.3 (2H, m,

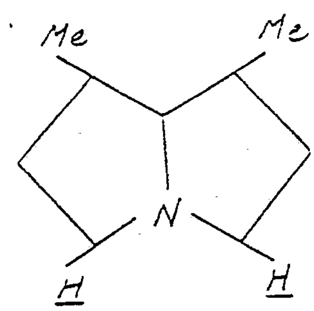

).

4.30 (2H, s, $CH_2NO_2$).

IR spectrum ($\nu_{max}^{neat}$) $cm^{-1}$ :
2930 (C-H), 1550, 1370 ($NO_2$).

d) <u>5-(1-Nitroethyl)-1-azabicyclo[3.3.0]octane</u>

Yield : 66.0%.
MS spectrum (EI/DI) m/z :
184 ($M^+$), 110 (base peak).

$^1$H-NMR spectrum ($CDCl_3$) δ ppm :

1.50 (3H, d, J = 6.8Hz, Me),

1.5 - 2.5 (8H, m,

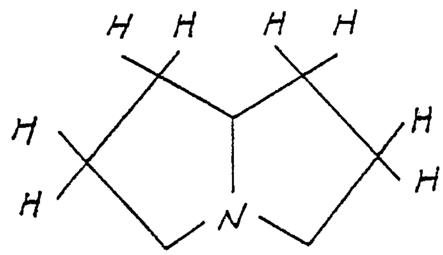

),

2.92 (4H, $A_2B_2$

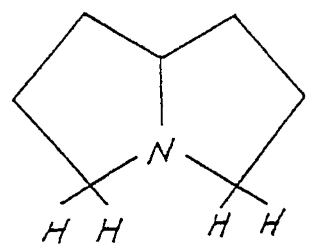

).

4.51 (1H, q, J = 6.8Hz, $CHNO_2$).

IR spectrum ($\nu_{max}^{neat}$) $cm^{-1}$ :
2930 (C-H), 1550, 1370 ($NO_2$).
e) <u>7-Cyano-1-azabicyclo[5.3.0]decane</u>
Yield : 41.1%.
MS spectrum (EI/DI) m/z :
164 ($M^+$), 138 (base peak).
$^1$H-NMR spectrum ($CDCl_3$) δ ppm :

1.5 - 2.0 (12H, m,

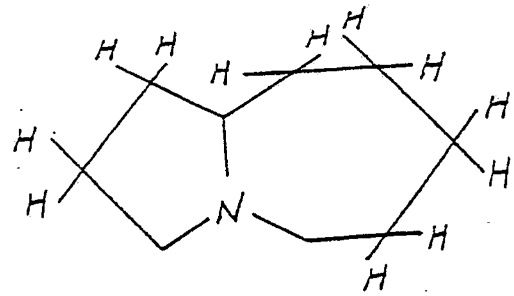

),

2.1 - 3.1 (4H, m,

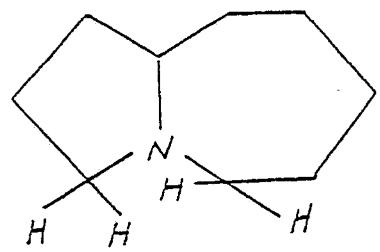

).

IR spectrum ($\nu_{max}^{neat}$) $cm^{-1}$ :
2950 (C-H), 2110 (CN).

Example 11
5-Aminomethyl-4,6-dimethyl-1-azabicyclo[3.3.0]octane

To a suspension of $LiAlH_4$ (69.2mg, 1.82mmol) in absolute ethyl ether (1.83ml) was added a solution of 5-cyano-4,6-dimethyl-1-azabicyclo[3.3.0]octane (100mg, 0.610mmol, Example 3) in absolute ethyl ether (0.73ml) under argon atmosphere with refluxing for 15 minutes. The mixture was further refluxed for 3hr. After cooled to 0°C, 15% sodium hydroxide solution (48.7mg) was gradually added to the reaction mixture, and the mixture was stirred, for a while, to obtain forming crystals through a filtration. The crystals were washed. While, the filtrate was concentrated in vacuo and the resulting crude product was purified by an alumina column chromatography (Merck No. 1097; elute : n-hexane/EtOAc = 50/1) to afford the desired compound which was combined with the aforesaid crystals (85.0mg, Yield : 82.8%).

MS spectrum (EI/DI) m/z :
168 ($M^+$), 138 (base peak).
$^1H$-NMR spectrum ($CDCl_3$) $\delta$ ppm :

1.04 (6H, d, J = 6.8Hz, 2 x Me),

1.5 - 2.0 (8H, m,

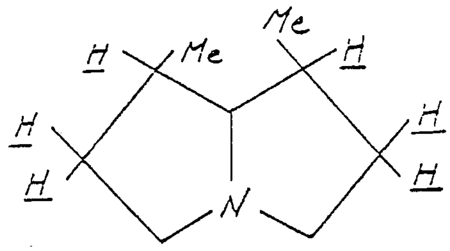

and $NH_2$),

2.4 - 2.6 (2H, m,

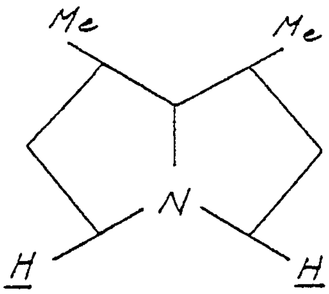

),

2.51 (2H, s, $\underline{CH_2}NH_2$)

3.1 - 3.2 (2H, m,

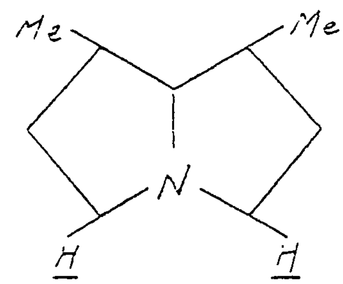

).

IR spectrum ($\nu_{max}^{neat}$) $cm^{-1}$ :
3375, 3300 (NH), 2950, 2860 (C-H).

Example 12
5-Methoxycarbonyl-2,8-dimethyl-1-azabicyclo[3.3.0]octane

A solution of 5-cyano-2,8-dimethyl-1-azabicyclo[3.3.0]octane (164mg, 1.00 mmol, Example 2) in absolute methanol (5.00ml) was cooled to 20° C under argon atmosphere and then gaseous hydrogen chloride was led into the solution for 30min. to saturate the same with hydrogen chloride. After stirring for 1hr at 20° C, the reaction mixture was poured into ice-water, and alkalized by 15% NaOH solution at 0 - 5° C. The resulting basic solution was extracted with methylene chloride. The extract was dried over $MgSO_4$ and concentrated in vacuo. The resulting crude product was purified by an alumina column chromatography (Merck No. 1097; elute : n-hexane/EtOAc = 50/1) to afford the desired compound (138mg, Yield : 70.0%).

MS spectrum (EI/DI) m/z :

197 ($M^+$) , 138 (base peak).
$^1$H-NMR spectrum ($CDCl_3$) $\delta$ ppm :

1.04 (6H, d, J = 6.8Hz, 2 x Me),

1.5 - 2.1 (8H, m,

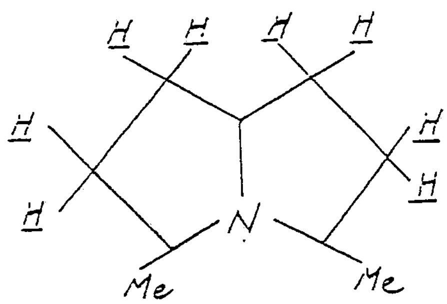

),

2.8 - 2.9 (2H, m,

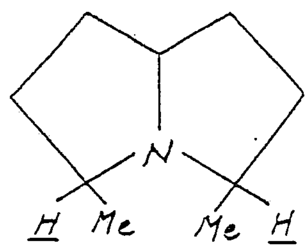

),

3.65 (3H, s, OMe).

IR spectrum ($\nu_{max}^{neat}$) $cm^{-1}$ :
2975 (C-H), 1730 (COOMe).

Example 13
5-Hydroxymethyl-2,8-dimethyl-1-azabicyclo[3.3.0]octane

To a suspension of $LiAlH_4$ (38.0mg, 1.00mmol) in absolute ethyl ether (5.00ml) was added a solution of 5-methoxycarbonyl-2,8-dimethyl-1-azabicyclo[3.3.0]octane (197mg, 1.00mmol, Example 12) in absolute ethyl ether (2.50ml) under argon atmosphere at 0 °C for 15 minutes. The reaction mixture was refluxed for 3hr, cooled to 0 °C and 15% sodium hydroxide solution was gradually added to cause decompose of excess $LiAlH_4$. Formed crystals was obtained through filtration and sufficiently washed with ethyl ether. The filtrate was concentrated in vacuo. The resulting crude product was purified by an alumina column chromatography (Merck No. 1097; elute : n-hexane/EtOAc = 50/1) to afford the desired compound (127mg, Yield : 75.0%).

MS spectrum (EI/DI) m/z :
169 ($M^+$), 138 (base peak).
$^1$H-NMR spectrum ($CDCl_3$) $\delta$ ppm :

1.08 (6H, d, J = 6.8Hz, 2 x Me),

1.5 - 2.1 (8H, m,

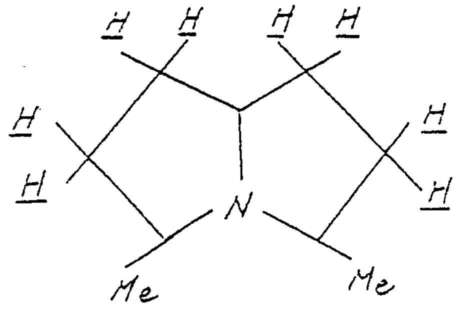

),

2.8 - 2.9 (2H, m,

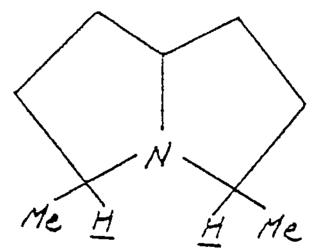

),

3.28 (2H, s, <u>CH$_2$</u>OH),

6.50 (1H, brs, OH).

IR spectrum ($\nu^{neat}_{max}$) cm$^{-1}$ :
3360 (OH), 2975 (C-H).

Example 14
5-Methoxymethyl-2,8-dimethyl-1-azabicyclo[3.3.0]octane

To a solution of 5-hydroxymethyl-2,8-dimethyl-1-azabicyclo[3.3.0]octane (169mg, 1.00mmol, Example 13) in toluene (5.00ml), was added a solution of methanesulfonylchloride (229mg, 2.00mmol) in toluene (5.00ml) at 0 - 5° C to stir the mixture for 12hr at 20° C. And then, the reaction mixture was added at 0° C to the NaOMe solution, which was prepared by the reaction of MeOH (48.0mg, 1.50mmol) and NaH (40.8mg, 1.70 mmol) in dioxane (5.00ml). The reaction mixture was refluxed for 2hr. The resulting reaction mixture was poured into ice-water and the resultant solution was alkalized by 15% NaOH solution. The resultant solution was extracted with methylene chloride and the extract was dried over $MgSO_4$ and concentrated in vacuo. The resulting crude product was purified by an alumina column chromatography (Merck No. 1097; elute : n-hexane/EtOA = 50/1) to afford the desired compound (100mg, Yield : 54.9%)

MS spectrum (EI/DI) m/z :
183 (M$^+$), 138 (base peak).
$^1$H-NMR spectrum ($CDCl_3$) $\delta$ ppm :

1.08 (6H, d, J = 6.8Hz, 2 x Me),

1.5 - 2.1 (8H, m,

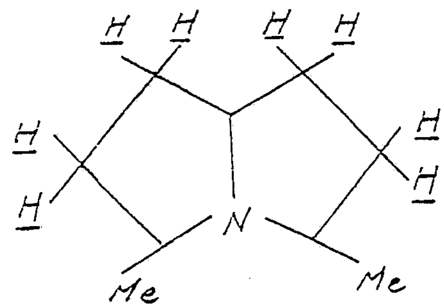

),

2.8 - 2.9 (2H, m,

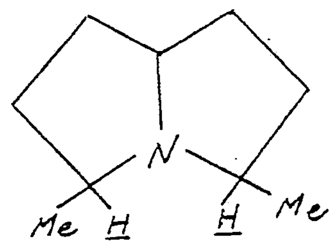

),

3.25 (3H, s, $OCH_3$),

3.50 (2H, s, $\underline{CH_2}O$).

IR spectrum ($\nu_{max}^{neat}$) $cm^{-1}$ :
2975 (C-H).

Example 15
5-Acetoxymethyl-2,8-dimethyl-1-azabicyclo[3.3.0]octane

To a solution of acetyl chloride (78.5mg, 1.00mmol) in toluene (5.00ml) was gradually added 5-hydroxymethyl-2,8-dimethyl-1-azabicyclo[3.3.0]octane (169mg, 1.00mmol, Example 13) at 0° C. The mixture was stirred at 20° C for 12hr. The reaction mixture was poured into ice-water and the solution was alkalized by 10% NaOH solution. The resulting solution was extracted with methylene chloride, and the extract was dried over $MgSO_4$ and concentrated in vacuo. The resulting crude product was purified by an alumina column chromatography (Merck No. 1097; elute : n-hexane/EtOAc = 50/1) to afford the desired compound (171 mg, Yield : 81 .1%).

MS spectrum (EI/DI) m/z :
211 ($M^+$), 138 (base peak).

$^1$H-NMR spectrum ($CDCl_3$) $\delta$ ppm :

1.08 (6H, d, J = 6.8Hz, 2 x Me),

1.5 - 2.1 (8H, m,

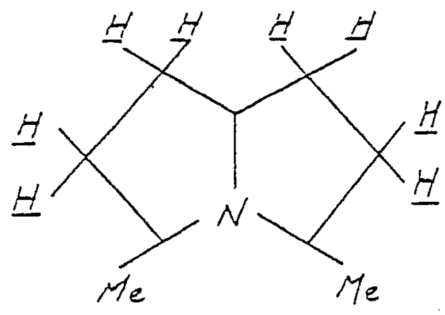

),

2.05 (3H, s, Me),

2.8 - 2.9 (2H, m,

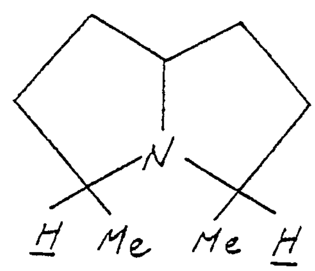

),

3.75 (2H, s, $CH_2O$).

IR spectrum ($\nu_{max}^{neat}$) $cm^{-1}$ :
2975 (C-H), 1735 (COO-).

Example 16

5-Acetamidemethyl-4,6-dimethyl-1-azabicyclo[3.3.0]octane

To a solution of 5-aminomethyl-4,6-dimethyl-1-azabicyclo[3.3.0]octane (168mg, 1.00mmol, Example 11) in toluene (5.00ml), was added a solution of acetyl chloride (75.8mg, 1.00mmol) in toluene (2.50ml) at 0 ° C. The mixture was stirred at 20 ° C for 12hr. The reaction mixture was poured into ice-water. The solution was alkalized by 10% NaOH solution. The resulting solution was extracted with methylene chloride, and the extract was dried over $MgSO_4$ and concentrated in vacuo. The resulting crude product was purified by an alumina column chromatography (Merck No. 1097; elute : n-hexane/EtOAc = 50/1) to afford the desired compound (164mg, Yield : 78.1%).

MS spectrum (EI/DI) m/z :
210 ($M^+$), 138 (base peak).

$^1$H-NMR spectrum ($CDCl_3$) δ ppm :

1.04 (6H, d, J = 6.8Hz, 2 x Me),

1.5 - 2.0 (6H, m,

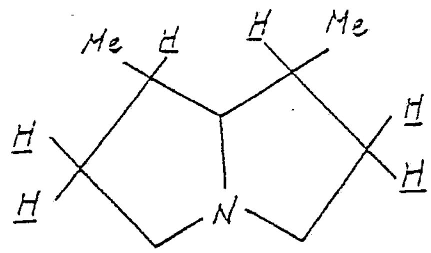

),

2.00 (3H, s, Me),

2.4 - 2.6 (2H, m,

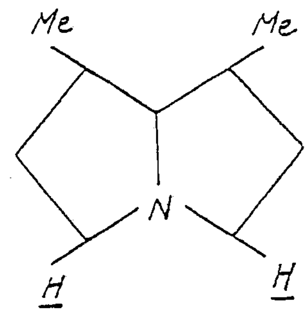

),

3.1 - 3.2 (2H, m,

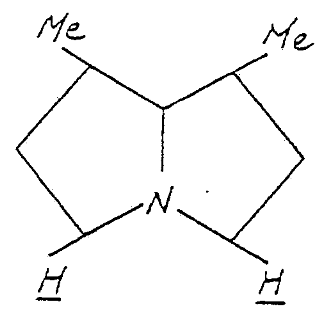

),

3.18 (2H, d, J = 6.0Hz, $\underline{CH_2}$NHAc),

6.50 (1H, br, NH).

IR spectrum ($\nu^{neat}_{max}$) $cm^{-1}$ :
2975 (C-H), 1670 (CONH).

Example 17
5-Carbamoyl-2,8-dimethyl-1-azabicyclo[3.3.0]octane

A solution of 5-methoxycarbonyl-2,8-dimethyl-1-azabicyclo[3.3.0]octane (197mg, 1.00mmol, Example 12) in methanol (5.00ml), was stirred under $NH_3$ atmosphere at 20°C for 24hr. The reaction mixture was concentrated *in vacuo* to afford the desired compound (180mg, Yield : 98.9%).

MS spectrum (EI/DI) m/z :
182 ($M^+$), 138 (base peak).

$^1$H-NMR($CDCl_3$) δ ppm

1.08 (6H, d, J = 6.8Hz, 2 x Me),
1.5 - 2.1 (8H, m,

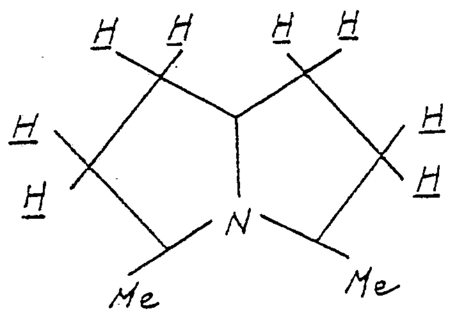

),

2.8 - 2.9 (2H, m,

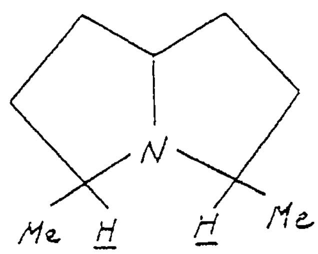

),

7.32 (2H, brs, $NH_2$).

IR spectrum ($\nu_{max}^{neat}$) $cm^{-1}$ :
2975 (C-H), 1630 ($CONH_2$).

Example 18
5-Acetyl-2,8-dimethyl-1-azabicyclo[3.3.0]octane

To a solution of 5-methoxycarbonyl-2,8-dimethyl-1-azabicyclo[3.3.0]octane (197mg, 1.00mmol, Example 12) in absolute ethyl ether (5.00ml), was added MeMgI (2mol/l ethyl ether solution, 0.500ml, 1.00mmol) at 0° C under argon atmosphere to stir at 0° C for 6hr and to reflux for 12hr. The reaction mixture was poured into 1.ON hydrochloric acid and the solution was stirred at 50° C for 2hr. After cooling, the solution was alkalized by 10% NaOH solution. The resulting solution was extracted with methylene chloride, the extract was dried over $MgSO_4$, and concentrated in vacuo. The resulting crude product was purified by an alumina column chromatography (Merck NO. 1097; elute : n-hexane/EtOAc = 50/1) to afford the desired compound (120mg, Yield : 66.3%).
MS spectrum (EI/DI) m/z :
181 ($M^+$), 138 (base peak).
$^1$H-NMR(CD3Cl) δ ppm

1.04 (6H, d, J = 6.8Hz, 2 x Me),
2.12 (3H, s, Me),

1.5 - 2.1 (8H, m,

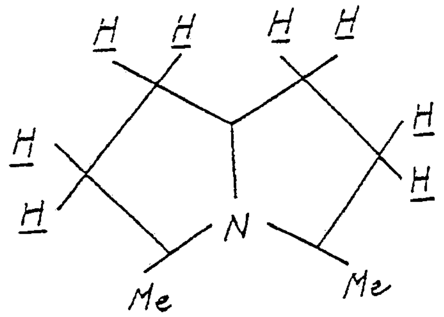

),

2.8 - 2.9 (2H, m,

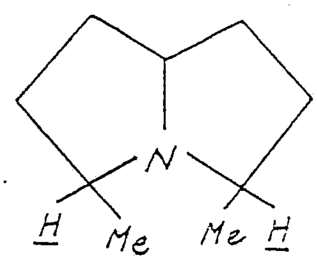

).

IR spectrum ($\nu_{max}^{neat}$) $cm^{-1}$ :
2975 (C-H), 1720 (C=O).

Example 19
5-Chloromethyl-2,8-dimethyl-1-azabicyclo[3.3.0]octane hydrochloride

To a solution of 5-hydroxymethyl-2,8-dimethyl-1-azabicyclo[3.3.0]octane (169mg, 1.00mmol, Example 13) in toluene (5.00ml), was added thionyl chloride (1190mg, 10.0mmol) at 5 - 10° C, and then the mixture was stirred at 70 - 80° C for 1hr. The toluene and excess thionyl chloride were distilled out in vacuo and the resulting residue was recrystallized from methylene/ethyl ether to afford the desired compound (salt) (180mg, Yield : 80.4%).

MS spectrum (EI/DI) m/z :
187 ($M^+$), 138 (base peak).
$^1$H-NMR(CD3Cl) δ ppm

1.04 (6H, d, J = 6.8Hz, 2 x Me),

1.9 - 2.7 (8H, m,

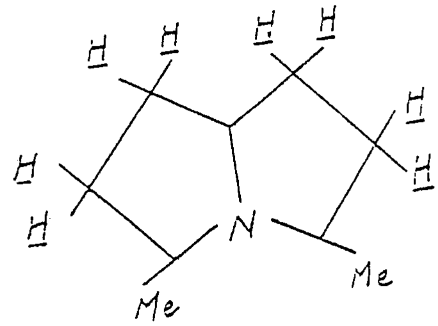

),

2.8 - 4.2 (3H, m,

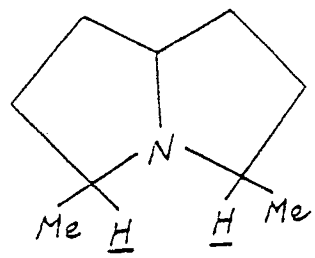

and HCl),

4.05 (2H, s, $CH_2Cl$).

## Claims

1. A 1-azabicyclo[m.n.O]alkane derivative of the formula

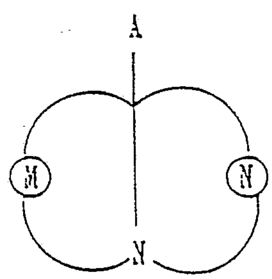

(I)

wherein M represents a Cm alkylene chain which may be substituted by 1-2m hydrocarbon groups, N represents Cn alkylene chain which may be substituted by 1 - 2n hydrocarbon groups, m and n are an integer of 3 - 5 (m > n), A represents a cyano radical, carboxylic acid group which has been esterified with an alcohol, a carboxylic acid group which has been amidized with a primary or secondary amine, an acyl group, or radical of $-C(A^1)(A^2)(A^3)$, in which at least one of $A^1$, $A^2$ and $A^3$ is a nitro radical, hydroxyl radical, hydroxyl group etherified with an alcohol or esterified with a carboxylic acid, unsubstituted or mono- or di-substituted amino group, acylated amino group or halogen atom, and the others of $A^1$, $A^2$ and $A^3$ are hydrogen or a hydrocarbon group,
and a salt thereof.

2. A 1-azabicyclo[m.n.O]alkane derivative or a salt thereof as claimed in Claim 1, which is selected from the group consisting of

a) 5-nitromethyl-2,8-dimethyl-1-azabicyclo[3.3.0]octane,
b) 5-nitromethyl-3,7-dimethyl-1-azabicyclo[3,3,0]octane,
c) 5-nitromethyl-4,6-dimethyl-1-azabicyclo[3.3.0]octane,
d) 5-(1-nitroethyl)-1-azabicyclo[3.3.0]octane,
e) 5-nitromethyl-1-azabicyclo[4.3.0]nonane,
f) 5-hydroxymethyl-2,8-dimethyl-1-azabicyclo[3.3.0]octane,
g) 5-methoxymethyl-2,8-dimethyl-1-azabicyclo[3.3.0]octane,
h) 5-acetoxymethly-2,8-dimethyl-1-azabicyclo[3.3.0]octane,
i) 5-aminomethyl-4,6-dimethyl-1-azabicyclo[3.3.0]octane,
j) 5-acetamidomethyl-4,6-dimetyl-1-azabicyclo[3.3.0]octane,
k) 5-chloromethyl-2,8-dimethyl-1-azabicyclo[3.3.0]octane,
l) 5-cyano-2,8-dimethyl-1-azabicyclo[3.3.0]octane,
m) 5-cyano-3,7-dimethyl-1-azabicyclo[3.3.0]octane,
n) 5-cyano-4,6-dimethyl-1-azabicyclo[3.3.0]octane,
o) 5-methoxycarbonyl-2,8-dimethly-1-azabicyclo[3.3.0]octane,
p) 5-carbamoyl-2,8-dimethyl-1-azabicyclo[3.3.0]octane,
q) 5-acetyl-2,8-dimethly-1-azabicyclo[3,3,0]octane, and
r) a salt thereof.

**3.** A process for the preparing of 1-azabicyclo[m.n.O]alkane derivatives of the formula

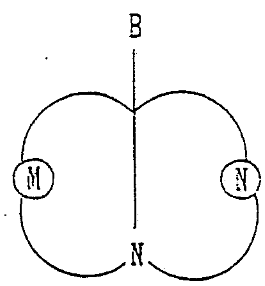

(V)

wherein M represents a Cm alkylene chain which may be substituted by 1 - 2m hydrocarbon groups, N represents Cn alkylene chain which may be substituted by 1 - 2n hydrocarbon groups, m and n are an integer of 3 - 5 (m > n), B represents a cyano radical or a group of -C($B^1$)($B^2$)($B^3$), in which at least one of $B^1$ , $B^2$ and $B^3$ is a nitro radical and the others are hydrogen atom or a hydrocarbon group,

and a salt thereof, which comprises reacting an α, ω -di-substituted alkanone of the formula

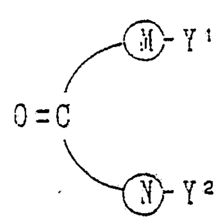

(II)

wherein M and N have the meanings as referred to, $Y^1$ and $Y^2$ represent a halogen atom or a group of $R^1$-$SO_3$-, in which $R^1$ is a hydrocarbon group,

with ammonia and a cyano compound of the formula

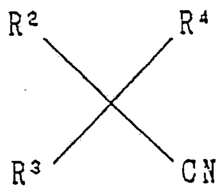

(III)

wherein $R^2$ and $R^3$ are same or different and represent hydrogen atom or a hydrocarbon group, respectively, and $R^4$ represents a hydroxyl radical or amino radical,

or a compound of the formula

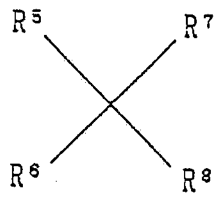

(IV)

wherein at least one of $R^5$, $R^6$, $R^7$ and $R^8$ is a nitro radical, and the others represent hydrogen atom or a hydrocarbon group,

and if necessary, converting resulting compound into the salt.